# EUROPEAN PATENT APPLICATION

(11) **EP 1 087 011 A2**
(43) Date of publication of application: **28.03.2001**
(21) Application number: 00203385.0
(22) Date of filing: 04.01.1995
(51) Int. Cl.: C12N 9/64, A61K 38/48

(54) **Methods for preventing degradation of protein c**

(30) Priority: 05.01.1994 US 177832
(62) Divisional of application: 95300042.9
(71) Applicant: ELI LILLY AND COMPANY, Indianapolis, Indiana 46285 (US)
(72) Inventor: Prouty, Walter Francis, Indianapolis, Indiana 46250 (US); Secnik, Josephine, Greenwood, Indiana 46143 (US)
(74) Representative: Denholm, Anna Marie

(57) **Abstract**

The present invention relates to methods to prevent or minimize autodegradation of activated protein C. The invention is best exemplified by performing the processing, purification and/or storage of the activated protein C at low pH, for example at about pH 6.3 to about pH 6.5. Autodegradation of aPC may also be minimized by incubating the aPC in 3 M urea (complete recovery of aPC activity is obtained after the removal of the denaturant) or by incubating the aPC in the presence of extreme salt concentrations. The present invention also comprises aPC formulations which maintain the aPC at low pH, in denaturant, or at extreme salt concentrations.

## Description

This invention relates to enzymology, particularly to methods for preventing degradation of activated protein C.

Protein C is a serine protease and naturally occurring anticoagulant that plays a role in the regulation of hemostasis by activating Factors Vₐ and VIIIₐ in the coagulation cascade. Human Protein C is made in vivo primarily in the liver as a single polypeptide of 461 amino acids. This precursor molecule undergoes multiple post-translational modifications including 1) cleavage of a 42 amino acid signal sequence; 2) proteolytic removal from the one chain zymogen of the lysine residue at position 155 and the arginine residue at position 156 to make the 2-chain form of the molecule, (i.e., a light chain of 155 amino acid residues attached through a disulfide bridge to the serine protease-containing heavy chain of 262 amino acid residues); 3) vitamin K-dependent carboxylation of nine glutamic acid residues clustered in the first 42 amino acids of the light chain, resulting in 9 gamma-carboxyglutamic acid residues; and 4) carbohydrate attachment at four sites (one in the light chain and three in the heavy chain). The heavy chain contains the well established serine protease triad of Asp 257, His 211 and Ser 360. Finally, the circulating 2-chain zymogen is activated in vivo by thrombin at a phospholipid surface in the presence of calcium ion. Activation results from removal of a dodecapeptide at the N-terminus of the heavy chain, producing activated protein C (aPC) possessing enzymatic activity.

In working with large quantities and high concentrations of aPC, a proteolytic clip at the lysine residue at position 308 of the heavy chain was observed. The sequence of the new N-terminus generated by this clip begins with Glu-Ala-Lys and yields an 111 amino acid fragment, termed "EAK fragment", from the C-terminal end of the heavy chain. The EAK fragment is not attached covalently to the light chain or the N-terminal portion of the heavy chain through disulfide bonds. The EAK fragment also contains the active site serine of the serine protease, but not the Asp or His residues. Thus, it has been discovered that protein C preparations with the EAK fragment have altered anticoagulant activity. The present invention comprises methods for preventing or minimizing the degradation of the Protein C molecule by maintaining the molecule at a lowered pH, in a denaturing agent, or in extremes of salt concentration.

For purposes of the present invention, as disclosed and claimed herein, the following terms are as defined below.
aPC - activated human Protein C.
APTT - activated partial thromboplastin time.
AU - amidolytic units.
BME - beta-mercaptoethanol.
CHES - 2[N-cyclohexylamino] ethane sulfonic acid.
EAK Fragment - the 111 amino acid fragment arising from a clip at position 308 of the heavy chain of protein C.
EDTA - ethylenediaminetetraacetic acid.
HEPES - N-2-hydroxyethyl piperazine-N'-2-ethane sulfonic acid.
HPC - human protein C zymogen.
MEA - 2-aminoethanol.
MES - 2-(N-morpholino)-ethanesulfonic acid.
Nascent protein - the polypeptide produced upon translation of an mRNA transcript, prior to any post-translational modifications. However, post-translational modifications such as gamma-carboxylation of glutamic acid residues and hydroxylation of aspartic acid residues may begin to occur before a protein is fully translated from an mRNA transcript.
Protein C Activity - any property of human protein C responsible for proteolytic, amidolytic, esterolytic, and biological (anticoagulant or profibrinolytic) activities. Methods for testing for protein C anticoagulant and amidolytic activity are well known in the art, i.e., see Grinnell et.al., 1987, Bio/Technoloqy 5:1189-1192.
rHPC - recombinantly produced human Protein C zymogen.
Zymogen - an enzymatically inactive precursor of a proteolytic enzyme. Protein C zymogen, as used herein, refers to secreted, inactive forms, whether one chain or two chain, of protein C.

All amino acid abbreviations used in this disclosure are those accepted by the United States Patent and Trademark Office as set forth in 37 C.F.R. §1.822(b)(2) (1990).

The present invention relates to methods to prevent or minimize autodegradation of activated protein C. The invention is best exemplified by performing the processing, purification and/or storage of the activated protein C at low pH, for example at about pH 6.3 to about pH 7.0. Autodegradation of aPC may also be minimized by incubating the aPC in 3 M urea (complete recovery of aPC activity is obtained after the removal of the denaturant) or by incubating the aPC in the presence of extreme concentrations of salt. For the purposes of the present disclosure, extreme salt concentration means salt concentrations above about 0.4 molar or below about 0.05 molar. The present invention also comprises aPC formulations which maintain the aPC at low pH, in denaturant, or at extreme salt concentrations.

The role of protein C in maintaining hemostasis has sparked a great interest in this compound as a therapeutic agent for a wide variety of vascular disorders. The production of high levels and high concentrations of human protein C at an industrial scale has uncovered the fact that the molecule can undergo autodegradation leading to decreased anticoagulant activity. Autodegradation of aPC results in the formation of an 111 amino acid fragment, termed "EAK fragment", from the C-terminal end of the heavy chain. The EAK fragment is not attached covalently to the light chain or the N-terminal portion of the heavy chain through disulfide bonds. The EAK fragment also contains the active site serine residue of the serine protease, but not the Asp or His residues. Thus, protein C preparations with the EAK fragment have altered anticoagulant activity due to the presence of the proteolytic clip.

To reduce or prevent the autodegradation that leads to the formation of the EAK fragment, the intact activated Protein C molecule can be maintained at a pH between about 6.3 and about 7.0. The molecule can be kept at a pH in this range throughout all purification and activation processes, as well as, in the final formulation solution. Many different buffer systems can be used to maintain the pH of the solution. Representative buffer systems include Tris-Acetate, Sodium Citrate, Citrate-Glycine and Sodium Phosphate. The skilled artisan will recognize that many other buffer systems are available which also can be used in the processes of the present invention.

Another aspect of the present invention relates to the prevention or reduction of the formation of the EAK fragment by maintaining the activated protein C molecule in a solution with an extreme salt concentration. For example, at pH 7.0 in a Sodium Phosphate buffer, the EAK fragment formation is minimal when there is no salt present in the buffer. However, at pH 7.0 in a Sodium Phosphate buffer, the EAK fragment formation is also minimal when there is a concentration of about 0.4 M sodium chloride present in the buffer. Between these two salt concentrations, EAK fragment formation occurs at variable rates; but the skilled artisan will recognize that maintaining salt concentrations below 0.05 M or salt concentrations above 0.4 M will most readily prevent or minimize EAK fragment formation. Other salt concentrations below 0.05 M (e.g., 0.01 M or 0.005 M) are preferable although the best pharmaceutical formulation occurs when the pH of the solution is maintained at about pH 7.0 with no salt added to the solution. The skilled artisan will recognize that many different salts may be used in pharmaceutical processing and formulations. Representative salts which may be used in the present invention include potassium chloride, calcium chloride and, most preferably, sodium chloride.

Yet another aspect of the present invention relates to preventing or reducing the autodegradation of activated protein C by performing purification and/or formulation in the presence of a denaturing agent. Many different denaturing agents can be used, but the most preferable agent is urea at concentrations of about 3 M.

The present invention is not limited by the methods by which the activated protein C may be produced. While it is most efficient to produce activated protein C molecules using recombinant DNA technology, recent advances in large-scale protein purification have allowed for the isolation of significant amounts of activated protein C from human serum. Obtaining such large amounts of protein C has allowed high concentrations of activated protein C to be processed at one time. As noted above, the inventors discovered that concentrations of activated protein C above the level of about 50 micrograms per milliliter demonstrated autodegradation of the activated protein C molecule with the concomitant drop in anticoagulant activity. Most importantly, the rate of autodegradation increases as the concentration of activated protein C is increased in a solution. On an industrial level, it is not efficient to run large scale purification processes at very low concentrations of protein.

In that the industrial and pharmaceutical utility of activated protein C production necessitates processing of the molecule at concentrations far exceeding 50 micrograms, the present invention allows the skilled artisan to produce large quantities of the product without significant loss in product activity. The formulations of the present invention further allow for the storage of the stable activated protein C solutions for a longer period of time than what was known in the prior art.

The skilled artisan will recognize that the methods of the present invention will allow those persons practicing the invention to create larger volumes of activated protein C in higher concentrations than were previously available without the fear of suffering product loss due to autodegradation. In addition, the stable pharmaceutical formulations of the present invention can easily be used to treat patients suffering from physical disorders as taught by Taylor, Jr. et. al., U.S. Patent No. 5,009,889, the entire teaching of which is herein incorporated by reference.

The following examples are provided as a means of illustrating the present invention and are not to be construed as a limitation thereon.

### Example 1

### Production of Human Protein C

Recombinant human protein C (rHPC) was produced in Human Kidney 293 cells by techniques well known to the skilled artisan such as those set forth in Yan, U.S. Patent No. 4,981,952, the entire teaching of which is herein incorporated by reference. The gene encoding human protein C is disclosed and claimed in Bang et al., U.S. Patent No. 4,775,624, the entire teaching of which is incorporated herein by reference. The plasmid used to express human protein C in 293 cells was plasmid pLPC which is disclosed in Bang et al., U.S. Patent No. 4,992,373, the entire teaching of which is incorporated herein by reference. The construction of plasmid pLPC is also described in European Patent Publication No. 0 445 939, and in Grinnell et al., 1987, Bio/Technology 5:1189-1192, the teachings of which are also incorporated herein by reference. Briefly, the plasmid was transfected into 293 cells, then stable transformants were identified, subcultured and grown in serum-free media. After fermentation, cell-free medium was obtained by microfiltration.

The human protein C was separated from the culture fluid by an adaptation of the techniques of Yan, U.S. Patent No. 4,981,952, the entire teaching of which is herein incorporated by reference. The clarified medium was made 4 mM in EDTA before it was absorbed to an anion exchange resin (Fast-Flow Q, Pharmacia). After washing with 4 column volumes of 20 mM Tris, 200 mM NaCl, pH 7.4 and 2 column volumes of 20 mM Tris, 150 mM NaCl, pH 7.4, the bound recombinant human protein C zymogen was eluted with 20 mM Tris, 150 mM NaCl, 10 mM CaCl₂, pH 7.4. The eluted protein was greater than 95% pure after elution as judged by SDS-polyacrylamide gel electrophoresis.

Further purification of the protein was accomplished by making the protein 3 M in NaCl followed by adsorption to a hydrophobic interaction resin (Toyopearl Phenyl 650M, TosoHaas) equilibrated in 20 mM Tris, 3 M NaCl, 10 mM CaCl₂, pH 7.4. After washing with 2 column volumes of equilibration buffer without CaCl₂, the recombinant human protein C was eluted with 20 mM Tris, pH 7.4. The eluted protein was prepared for activation by removal of residual calcium. The recombinant human protein C was passed over a metal affinity column (Chelex-100, Bio-Rad) to remove calcium and again bound to an anion exchanger (Fast Flow Q, Pharmacia). Both of these columns were arranged in series and equilibrated in 20 mM Tris, 150 mM NaCl, 5 mM EDTA, pH 7.4. Following loading of the protein, the Chelex-100 column was washed with one column volume of the same buffer before disconnecting it from the series. The anion exchange column was washed with 3 column volumes of equilibration buffer before eluting the protein with 0.4 M NaCl, 20 mM Tris-acetate, pH 6.5. Protein concentrations of recombinant human protein C and recombinant activated protein C solutions were measured by UV 280 nm extinction E^{0.1%=}1.85 or 1.95, respectively.

### Example 2

### Activation of recombinant human Protein C

Bovine thrombin was coupled to Activated CH-Sepharose 4B (Pharmacia) in the presence of 50 mM HEPES, pH 7.5 at 4°C. The coupling reaction was done on resin already packed into a column using approximately 5000 units thrombin/ml resin. The thrombin solution was circulated through the column for approximately 3 hours before adding MEA to a concentration of 0.6 ml/l of circulating solution. The MEA-containing solution was circulated for an additional 10-12 hours to assure complete blockage of the unreacted amines on the resin. Following blocking, the thrombin-coupled resin was washed with 10 column volumes of 1 M NaCl, 20 mM Tris, pH 6.5 to remove all non-specifically bound protein, and was used in activation reactions after equilibrating in activation buffer.

Purified rHPC was made 5mM in EDTA (to chelate any residual calcium) and diluted to a concentration of 2 mg/ml with 20 mM Tris, pH 7.4 or 20 mM Tris-acetate, pH 6.5. This material was passed through a thrombin column equilibrated at 37°C with 50 mM NaCl and either 20 mM Tris pH 7.4 or 20 mM Tris-acetate pH 6.5. The flow rate was adjusted to allow for approximately 20 min. of contact time between the rHPC and thrombin resin. The effluent was collected and immediately assayed for amidolytic activity. If the material did not have a specific activity (amidolytic) comparable to an established standard of aPC, it was recycled over the thrombin column to activate the rHPC to completion. This was followed by 1:1 dilution of the material with 20 mM buffer as above, with a pH of either 7.4 or 6.5 to keep the aPC at lower concentrations while it awaited the next processing step.

Removal of leached thrombin from the aPC material was accomplished by binding the aPC to an anion exchange resin (Fast Flow Q, Pharmacia) equilibrated in activation buffer (either 20 mM Tris, pH 7.4 or 20 mM Tris-acetate, pH 6.5) with 150 mM NaCl. Thrombin does not interact with the anion exchange resin under these conditions, but passes through the column into the sample application effluent. Once the aPC is loaded onto the column, a 2-6 column volume wash with 20 mM equilibration buffer is done before eluting the bound aPC with a step elution using 0.4 M NaCl in either 5 mM Tris-acetate, pH 6.5 or 20 mM Tris, pH 7.4. Higher volume washes of the column facilitated more complete removal of the dodecapeptide. The material eluted from this column was stored either in a frozen solution (-20°C) or as a lyophilized powder.

The amidolytic activity (AU) of aPC was determined by release of p-nitroanaline from the synthetic substrate H-D-Phe-Pip-Arg-p-nitroanilide (S-2238) purchased from Kabi Vitrum using a Beckman DU-7400 diode array spectrophotometer. One unit of activated protein C was defined as the amount of enzyme required for the release of 1 µmol of p-nitroaniline in 1 min. at 25°C, pH 7.4, using an extinction coefficient for p-nitroaniline at 405 nm of 9620 M⁻¹cm⁻¹.

The anticoagulant activity of activated protein C was determined by measuring the prolongation of the clotting time in the activated partial thromboplastin time (APTT) clotting assay. A standard curve was prepared in dilution buffer (1 mg/ml radioimmunoassay grade BSA, 20 mM Tris, pH 7.4, 150 mM NaCl, 0.02% NaN₃) ranging in protein C concentration from 125-1000 ng/ml, while samples were prepared at several dilutions in this concentration range. To each sample cuvette, 50 µl of cold horse plasma and 50 µl of reconstituted activated partial thromboplastin time reagent (APTT Reagent, Sigma) were added and incubated at 37 °C for 5 min. After incubation, 50 µl of the appropriate samples or standards were added to each cuvette. Dilution buffer was used in place of sample or standard to determine basal clotting time. The timer of the fibrometer (CoA Screener Hemostasis Analyzer, American Labor) was started immediately after the addition of 50 µl 37°C 30 mM CaCl₂ to each sample or standard. Activated Protein C concentration in samples are calculated from the linear regression equation of the standard curve. Clotting times reported here are the average of a minimum of three replicates, including standard curve samples.

To prepare samples for comparative studies, complete autodegradation of aPC (7.3 mg/ml in 20 mM Tris, 150 mM NaCl) was achieved after 44 hours of incubation at 25°C or after concentration on anion exchange resin (FFQ, Pharmacia), pH 7.4 at 4°C. Amidolytic activity assays, HPLC, N-terminal sequencing and SDS-PAGE were performed on samples to confirm and quantitate the amount of autodegradation and the sites of cleavage on the amino acid sequence.

### Example 3

### Activated Protein C Stabilization Assays

The effect of pH on the activity of aPC was examined by monitoring AU. A three buffer system was used to establish pH conditions in the range of 6-9.3 (in 0.3 pH unit increments) by using 50 mM each of MES (pH 5.5-7), HEPES (pH 6.8-8.2), and CHES (pH 8.6-10). The aPC was diluted to a final concentration of 0.4 mg/ml with the appropriate pH buffer and incubated at this concentration prior to measurement of activity. Samples were assayed at both the initial time and after 30 hours at 4°C using amidolytic assays done at the incubation pH. These measurements showed a bell-shaped pH dependence for amidolytic activity. The results of these assays are set forth below in Table I.

**Table I**

| Amidolytic Rates (IU/mg aPC) | | |
|---|---|---|
| pH | 0 Hours | 30 Hours |
| 6.0 | 1.63 | 1.58 |
| 6.6 | 4.00 | 4.63 |
| 7.2 | 9.16 | 6.68 |
| 7.8 | 11.26 | 9.26 |
| 8.4 | 7.05 | 5.16 |
| 9.0 | 3.53 | 3.53 |

The activity maximum for aPC was pH 7.4, while the extreme pH values of 6 and 9.3 showed greatly reduced activity. Although aPC appears to retain some amidolytic activity at pH extremes, this data suggests that autodegradation is reduced by avoiding pH conditions in which the aPC had maximal activity.

To determine if the amidolytic activity pH dependence of aPC was a function of EAK fragment formation, aPC samples were incubated at pH 6.0, 7.5, and 9.0, 1.5 mg/ml at 4°C for 18 hrs. The amount of EAK formed was measured by integration of the area under the EAK HPLC peak as well as qualitative observation of the EAR band on SDS-PAGE. These data showed no increase in EAK formation during the course of the incubation at pH 6.0, while there was approximately a 30% increase at pH 7.5 and a 50% increase at pH 9.0. Despite elevated levels of EAK fragment in the pH 7.5 and 9.0 samples, the preparation still had high AU activity when assayed at pH 7.4. Thus, generation of the EAK fragment is not associated necessarily with a loss of amidolytic activity. Rather, the EAK fragment must stay associated enough with the rest of the aPC heavy chain to maintain serine protease functionality. If anything, higher EAK fragment content is associated with higher amidolytic activity.

The EAK fragment contains the active site serine (residue 360) of the catalytic triad (including His 211 and Asp 257 found in the N-terminal portion of the heavy chain). Therefore, if the EAK fragment is not covalently attached to the rest of the heavy chain, this proteolytic clip may result in reduced enzymatic activity. To address the impact of various amounts of EAK on anticoagulant activity, several different lots of aPC were prepared as described in Examples 1 and 2, supra. Amidolytic assays were done with substrate concentrations ranging from 22-198 uM #S-2238, aPC concentrations ranging from 1.6-3.3 nM (75-150 ng/ml) 20 mM Tris, pH 7.4, 150 mM NaCl. Results of this assay are set forth below in Table II.

**Table II**

| Percent EAK content versus specific activity measured by amidolytic and anticoagulant activities | | | |
|---|---|---|---|
| Sample Number | % EAK | Specific Activity | |
| | | AU/mg | APTT/mg |
| 1 | 3 | 1.13 | 2.11 |
| 2 | 19 | 1.35 | 1.7 |
| 3 | 35 | 1.52 | 1.37 |
| 4 | 51 | 1.64 | 1.00 |
| 5 | 67 | 1.68 | 0.78 |

The pH dependence of degradation was exploited to generate aPC samples which contained various amounts of EAK. These samples were prepared as described in Examples 1 and 2, supra. Samples with varying content of EAK fragment were compared to intact aPC (<3% EAK fragment). Amidolytic kinetic parameters, including Km, Kcat, and Vmax values, were measured for each of these lots at three different enzyme concentrations, and are summarized in Table III.

**Table III**

| Kinetic Data | | | | |
|---|---|---|---|---|
| Sample No. (% EAK) | [aPC] ng/ml | Km (mM) | Vmax (umol/ml/min) | Kcat (1/sec) |
| 1 (20%) | 150 | 0.12 | 9102 | 7 |
| 1 | 113 | 0.12 | 8634 | 6.6 |
| 1 | 75 | 0.19 | 8713 | 6.7 |
| 2 (67%) | 150 | 0.18 | 10672 | 8.2 |
| 2 | 113 | 0.18 | 9729 | 7.5 |
| 2 | 75 | 0.18 | 9930 | 7.6 |
| 3 (100%) | 150 | 0.16 | 13142 | 10.1 |
| 3 | 113 | 0.14 | 2337 | 1.8 |
| 3 | 75 | 0.18 | 316 | 0.2 |

Km values for the three aPC samples appeared the same within experimental variation and show an average value of 0.16 mM substrate. This suggests that affinity for the S-2238 substrate is not disrupted in degraded aPC. Surprisingly, degraded material still had AU activity essentially similar to that of intact aPC.

Activated protein C can have intact amidolytic functionality versus a tripeptide substrate (#S-2238) even with a high EAK fragment content. In vitro anticoagulant activity can be measured in the APTT assay. Unexpectedly, high AU activity did not correlate to anticoagulant activity. While AU activity increased with increasing EAK content, APTT activity decreased with increasing EAK content.

The effect of salt concentration on EAK fragment formation and activated protein C stability was studied by incubating samples of activated protein C at various pH levels and salt concentrations, then measuring the percentage of EAK fragment formation per hour. Protein C concentrations in the assay were between 4-5 milligrams per milliliter. All assays were performed in 5 to 20 mM phosphate buffer. Sodium chloride was used as a salt (when salt was present) and all assays were run at 25°C. The percentage of EAK fragment formation per hour was measured by HPLC integration. Results of these assays are set forth below in Table IV.

**Table IV**

| Effect of Salt Concentration on EAK fragment formation | | |
|---|---|---|
| pH | salt concentration | % EAK formed/hour |
| 6.4 | 400 mM | .094 |
| 7.0 | 400 mM | .145 |
| 6.4 | 50 mM | .292 |
| 7.0 | 50 mM | .365 |
| 6.4 | 0 | .038 |
| 7.0 | 0 | .092 |

## Claims

1. A process for minimizing the degradation of activated protein C said process comprising maintaining said activated protein C in a solution containing a denaturing agent.

2. A process for minimizing the degradation of activated protein C said process comprising maintaining said activated protein C in a solution with a pH between about 6.3 and about 7.0.

3. The process of Claim 2 wherein said solution has a salt concentration of below about 0.050 M or above about 0.40 M.

4. The process of Claim 3 wherein said solution has a salt concentration below about 0.050 M.

5. The process of Claim 3 wherein said solution has a salt concentration below about 0.010 M.

6. The process at Claim 3 wherein the salt in the solution is sodium chloride.

7. A stable pharmaceutical formulation comprising activated protein C in a solution with a pH between about 6.3 and about 7.0.

8. The pharmaceutical formulation of Claim 7 which further comprises salt in a concentration of below about 0.050 M or above about 0.4 M.

9. The pharmaceutical formulation of Claim 8 wherein the salt concentration is below about 0.05 M.

10. The pharmaceutical formulation of Claim 7 wherein the buffer is selected from the group Consisting of Tris-Acetate, Sodium Citrate, Citrate-Glycine and Sodium Phosphate.
